# EUROPEAN PATENT APPLICATION

(11) **EP 4 501 453 A1**
(43) Date of publication of application: **05.02.2025**
(21) Application number: 22933087.3
(22) Date of filing: 04.11.2022
(51) Int. Cl.: B01J 35/10, B01J 21/04, B01J 21/06, B01J 23/755

(54) **COMPOSITE OXIDE, PREPARATION METHOD FOR COMPOSITE OXIDE, HYDROGENATION CATALYST AND USE THEREOF**

(30) Priority: 24.03.2022 CN 202210303144
(71) Applicant: CHINA PETROLEUM & CHEMICAL CORPORATION, Chaoyang District Beijing 100728 (CN); Sinopec (Beijing) Research Institute of Chemical Industry Co., Ltd., Beijing 100013 (CN)
(72) Inventor: DU, Zhou, Beijing 100013 (CN); LIU, Yanhui, Beijing 100013 (CN); ZHANG, Fuchun, Beijing 100013 (CN); LIU, Zongyu, Beijing 100013 (CN); JI, Jing, Beijing 100013 (CN); REN, Yumei, Beijing 100013 (CN); YANG, Guang, Beijing 100013 (CN)
(74) Representative: Hoffmann Eitle
(86) International application number: PCT/CN2022/129813
(87) International publication number: WO 2023/179036

(57) **Abstract**

The present invention relates to a composite oxide, the composite oxide comprising 60-95 wt% of aluminum oxide and 5-40 wt% of titanium dioxide. The specific surface area of the composite oxide determined by means of BET method is expressed as X m²/g; the average pore diameter of the composite oxide determined by means of nitrogen adsorption isothermal curve method is expressed as Y nm, wherein the ratio of X to Y is 5-30; and by means of the determination of X-ray diffraction method, titanium dioxide in an anatase crystalline phase in the composite oxide accounts for 95-100 wt% of the total titanium dioxide, wherein X is in the range of 50-200, preferably X is in the range of 60-180, more preferably in the range of 80-150, and Y is in the range of 5-25 nm. The present invention further provides a hydrogenation catalyst comprising the composite oxide, which catalyst shows a very high vinyl acetylene conversion rate and high 1,3-butadiene selectivity.

## Description

### Technical field

The invention relates to a composite oxide, a preparation method of the composite oxide, a hydrogenation catalyst, and its application. Specifically, the hydrogenation catalyst can be used to remove alkynes in a C₄ fraction via hydrogenation and increase the production of butadiene.

### Background art

C₄ fraction refers to a mixture of various alkanes, olefins, diolefins and alkynes, which contain four carbon atoms. It is mainly derived from a refinery gas produced in petroleum refining processes and a byproduct of the cracking of petroleum hydrocarbons to ethylene. A cracking C₄ stream may contain saturated hydrocarbons and unsaturated hydrocarbons such as n-butane, isobutane, 1-butene, trans-2-butene, cis-2-butene, isobutene, 1,2-butadiene, 1,3-butadiene, methyl acetylene, ethyl acetylene, vinyl acetylene, and the like, and is mainly used in industry to produce 1,3-butadiene, isobutylene and 1-butene.

Cracking C₄ fractions, by-products of high-temperature cracking of hydrocarbons to produce ethylene, usually contain 40% - 60% by mass of butadiene. Butadiene is an important monomer in synthetic rubber industry. Solvent extraction methods, such as acetonitrile method, N-methylpyrrolidone method and dimethylformamide method, are usually used to extract butadiene from the cracking C₄ fractions. At present, these methods can basically meet the requirements for butadiene purity.

Due to the influence of factors such as cracking severity and cracking technology, the alkyne content in the cracking carbon four fractions gradually increases, resulting in an increase in the loss of butadiene and an increase in energy consumption during the extraction process. At the same time, as the organic synthesis industrial technology progresses, restrictions on the alkyne content in butadiene have become more stringent. These factors have led to poor economics of butadiene extraction units. In the process of extracting butadiene, partially recovering butadiene after selectively hydrogenating alkynes can not only achieve the purpose of turning waste into treasure, but also play an important role in reducing alkyne emissions and preventing environmental pollution.

Currently, alkynes in mixed carbon four hydrocarbon fractions can be removed by catalytic selective hydrogenation. The catalysts used tend to primarily use noble metal catalysts represented by palladium, platinum, silver, etc., and secondarily non-noble metal catalysts represented by copper and nickel.

In the selective hydrogenation of alkynes in hydrocarbon streams, the catalysts and reaction conditions selected are different, depending on the composition of the raw materials and the target products. In addition to having a high hydrogenation activity, a good selective hydrogenation catalyst should have a good stability, that is, the catalyst must have an ability to resist impurities and colloids so as to extend the life of the catalyst. Therefore, the carrier is required to have a lower acidity, a smaller specific surface area and a larger pore size. In addition, adding some additives during the preparation of the catalyst can also extend the service life of the catalyst.

As noble metal catalysts, palladium catalysts supported on carriers (usually alumina) are generally used, with other cocatalyst components, such as gold, silver, chromium, copper, iron, rhodium, lithium, potassium, lead, or zinc being added. Noble metal catalysts have a good low-temperature activity and mild reaction conditions, but they suffer from ease of loss of their active components, expensive price, not ease to regenerate, and slightly poor hydrogenation selectivity. Non-noble metal catalysts need to react at higher temperatures and have harsh hydrogenation conditions, but they are simple to prepare and easy to regenerate repeatedly, and have relatively low costs, so they are still worthy to be researched and developed to some extent. During this type of hydrogenation reaction, semi-hydrogenated free radicals adsorbed on the catalyst react with adjacent alkynes or dienes to form a viscous polymer (commonly known as green oil), which is mainly composed of C₆ and above compounds, and because the polymer covers the surface of the catalyst, blocking the micropores on the surface of the catalyst, the activity of the catalyst will decrease and the service life of the catalyst will be affected. Especially for conjugated dienes (e.g., 1,3-butadiene), their polymerization reaction proceeds more easily, thereby deactivating the catalyst in a short time, so that the catalyst must be regenerated frequently to be reused.

In application research in the field of selective hydrogenation of C₄ alkynes, it has been realized that for raw materials with high concentration of alkynes, it is safer and more efficient to use nickel-based selective hydrogenation catalysts, although the nickel-based selective hydrogenation catalysts are inferior to noble metal palladium-based catalysts in terms of activity and selectivity so that their application is limited. Therefore, for non-noble metal catalysts for the selective hydrogenation of alkynes, it is necessary to further improve the activity and selectivity of the catalysts, conduct research on countermeasures for the green oil problem during the use of the catalysts, and extend the service life of the catalysts. For the selective hydrogenation of alkynes, the use of non-noble metal hydrogenation catalysts can improve the catalysts' resistance to impurity poisoning and stability, which has economic value.

According to research in the field of photocatalysts, the structure and shape of catalytic materials based on TiO₂ can greatly affect the light absorption efficiency of the materials. Coral reefs have excellent light-absorbing and reflective structures, providing a three-dimensional (3D) environment for many plants and animals on the seafloor, and can absorb small particles, thereby supporting approximately a quarter of all known marine life. Coral-like 3D layered structures can have higher specific surface areas, resulting in more active sites and stronger light-harvesting capabilities, and have been shown to have better photocatalytic performance than other structures. The performance of TiO₂-supported catalysts can be improved by using specific catalytic materials with a morphology similar to coral reefs.

### Disclosures of the invention

In view of the shortcomings of the prior art, the objective of the present invention is to provide a non-noble metal selective hydrogenation catalyst with high low-temperature activity, high selectivity and high stability. Compared with noble metal selective hydrogenation catalysts for alkyne removal, the catalyst of the present disclosure can save investment cost by more than 80% and has better resistance to impurity poisoning and raw material adaptability.

In a first aspect, the present invention provides a composite oxide comprising aluminum oxide and titanium dioxide, wherein the composite oxide has a specific surface area expressed as X m²/g and an average pore diameter expressed as Y nm, with a ratio of X to Y being from 5 to 30, and wherein in the composite oxide, titanium dioxide in an anatase crystal phase accounts for 95 wt% to 100 wt% of the total titanium dioxide. Preferably, the ratio of X to Y is from 5 to 15. Further preferably, the ratio of X to Y is from 5 to 10. Preferably, in the composite oxide, titanium dioxide in the anatase crystal phase accounts for 96 wt% to 100wt% of the total titanium dioxide. Preferably, in the composite oxide, titanium dioxide in the anatase crystal phase accounts for 97 wt% to 100 wt% of the total titanium dioxide. Preferably, in the composite oxide, titanium dioxide in the anatase crystal phase accounts for 98 wt% to 100 wt% of the total titanium dioxide. Preferably, in the composite oxide, titanium dioxide in the anatase crystal phase accounts for 99 wt% to 100 wt% of the total titanium dioxide.

In the present invention, the proportion of titanium dioxide in the anatase crystal phase accounting for the total titanium dioxide can be measured through X-ray diffraction analysis.

In the present invention, the specific surface area of the composite oxide can be measured by the BET method.

In the present invention, the average pore diameter of the composite oxide can be measured by the nitrogen adsorption isotherms method.

In some embodiments of the invention, the diffraction peak area representing the anatase-type titanium dioxide crystalline structure, measured by X-ray diffraction analysis, accounts for 95 wt % to 100 wt % of the diffraction peak area of all titanium dioxide crystal structures.

In some embodiments of the invention, the diffraction peak area representing the anatase-type titanium dioxide crystalline structure, measured by X-ray diffraction analysis, accounts for 96 wt % to 100 wt % of the diffraction peak area of all titanium dioxide crystal structures.

In some embodiments of the invention, the diffraction peak area representing the anatase-type titanium dioxide crystalline structure, measured by X-ray diffraction analysis, accounts for 97 wt % to 100 wt % of the diffraction peak area of all titanium dioxide crystal structures.

In some embodiments of the invention, the diffraction peak area representing the anatase-type titanium dioxide crystalline structure, measured by X-ray diffraction analysis, accounts for 98 wt % to 100 wt % of the diffraction peak area of all titanium dioxide crystal structures.

In some embodiments of the invention, the composite oxide has a pore volume of Z mL/g, as measured by using a P/Po single-point desorption curve, and a ratio of X to Z is from 220 to 400, and preferably from 250 to 350. In some embodiments, the ratio of X to Z is 250, 260, 270, 280, 290, 300, 310, 320, 330, 340, or 350, or in a range between any two thereof.

In some embodiments of the invention, X is from 90 to 150. In some embodiments, X is 90, 100, 110, 120, 130, 140, or 150, or in a range between any two thereof.

In some embodiments of the invention, Y is from 9 to 20, and preferably from 12 to 16. In some embodiments, Y is 12, 13, 14, 15, or 16, or in a range between any two thereof.

In some embodiments of the invention, in the composite oxide, the proportion of pores with a pore diameter ranging from 10 to 20 nm accounting for all pores is at least 85 % by volume.

In some embodiments of the invention, Z is from 0.3 to 0.5, and preferably from 0.3 to 0.4. In some embodiments, Z is 0.31, 0.32, 0.33, 0.34, 0.35, 0.36, 0.37, 0.38, 0.39, or 0.40, or in a range between any two thereof.

In some embodiments of the invention, the composite oxide comprises 5 wt% to 21 wt% of titanium dioxide, based on the total weight of the composite oxide. In some embodiments, the composite oxide comprises the titanium dioxide at an amount of 5 wt%, 6 wt%, 8 wt%, 10 wt%, 12 wt%, 15 wt%, 18 wt%, 20 wt%, or 21 wt%, or in a range between any two thereof.

In some embodiments of the invention, the composite oxide comprises 79 wt% to 95 wt% of aluminum oxide, based on the total weight of the composite oxide.

In some embodiments of the invention, the composite oxide has a coral thicket-like 3D layered structure. The coral thicket-like 3D layered structure can have a higher specific surface area, resulting in more active sites and stronger light-harvesting capabilities.

In a second aspect, the present invention provides a method for preparing a composite oxide, through which an alumina-titanium dioxide composite oxide with a 3D microscopic morphology of coral thicket can be obtained. The preparation method according to the invention comprises the following steps:
I, dissolving a soluble aluminum source in water to form an aluminum source solution, dissolving a titanium source in an acid solution to form a titanium source solution, and mixing an ammonium salt and an alkali liquid to form a mixed alkali solution;
II, (a) adding the titanium source solution and the mixed alkali solution to the aluminum source solution, and maintaining a resulting mixed solution at a first pH value for a first period of time; (b) adding an additional amount of the mixed alkali solution to the mixed solution, and maintaining a resulting mixed solution at a second pH value for a second period of time; (c) adding an additional amount of the titanium source solution to the mixed solution, and maintaining a resulting mixed solution at a third pH value for a third period of time;
III, after step II(c), enhancing the temperature of the mixed solution and maintaining that temperature for a fourth period of time to obtain a precipitate; and
IV, drying and calcining the precipitate to obtain a composite oxide comprising alumina and titanium dioxide, with washing and filtering being preferably conducted (to remove unnecessary impurities) prior to the drying.

The composite oxide so obtained has the composition and structural characteristics described in the first aspect of the invention.

In some embodiments of the method of the invention, in step II, the first pH value is less than 5, and preferably in a range of from 3 to 4. In some embodiments of the method of the invention, in step II, the second pH value is greater than 8.5, and preferably in a range of from 9 to 10. In some embodiments of the method of the invention, in step II, the third pH value is greater than 7 and less than 9, and preferably in a range of from 7.5 to 8.5.

In some embodiments of the method of the invention, in step II, each of the first, second and third periods of time is from 5 to 20 minutes, and preferably from 10 to 15 minutes.

In some embodiments of the method of the invention, in step III, the fourth period of time is from 20 to 60 minutes.

In some embodiments of the method of the invention, in step II, the operating temperature is from 25°C to 60°C, and preferably from 50°C to 60°C.

In some embodiments of the method of the invention, in step III, the temperature is enhanced to 80°C to 150°C, and preferably 80°C to 100°C. In some embodiments, in step III, the temperature is enhanced to 80°C, 85°C, 90°C, 92°C, or 95°C.

In some embodiments of the method of the invention, in step IV, the drying temperature is in a range of from 110°C to 130°C. The drying time is from 4 to 12 hours, and preferably from 6 to 10 hours.

In some embodiments of the method of the invention, in step IV, the calcination temperature is in a range of from 800°C to 1000°C. In some embodiments, in step IV, the calcination temperature is 800°C, 850°C, 900°C, or 950°C, or in a range between any two thereof. The calcination time is from 4 to 12 hours, and preferably from 5 to 8 hours.

In some embodiments of the method of the invention, the aluminum source solution has an aluminum concentration of 0.5 to 2.5 mol/L. The aluminum source is a soluble aluminum salt, for example, one or more of aluminum sulfate, aluminum chloride, aluminum nitrate, and organic aluminums such as aluminum isopropoxide, aluminum sec-butoxide, aluminum monoacetate, and aluminum diacetate.

In some embodiments of the method of the invention, the titanium source solution has a titanium concentration of 0.2 to 1.2 mol/L. The titanium source is a soluble titanium source, which can be selected from titanium salts such as titanium acetate, titanium chloride, titanium sulfate, and titanium nitrate, and titanate esters such as tetraethyl titanate, tetra-n-propyl titanate, tetraisopropyl titanate and tetrabutyl titanate.

In some embodiments of the method of the invention, the mixed alkali solution has an ammonium salt concentration of 0.1 to 0.3 mol/L. The ammonium salt may be one or more selected from ammonium bicarbonate, ammonium carbonate, and organic ammonium salts.

In some embodiments of the method of the invention, the alkali liquid has an alkali concentration of 0.2 to 0.4 mol/L. The alkali liquid can be one or more selected from ammonia, sodium hydroxide, potassium hydroxide, and organic bases such as triethylamine, N,N-dimethylaniline, pyridine and quinoline.

In some embodiments of the method of the invention, the acid solution can be one or more selected from sulfuric acid, nitric acid, hydrochloric acid, and organic acids such as formic acid, acetic acid, citric acid, and oxalic acid.

In some embodiments of the method of the invention, ratios of the reactants charged in steps II(a), 11(b) and II(c) are as follows.

A ratio of the titanium source solution charged in steps II(a) and II(c) can be in a range of 1:4 to 2:3. Alternatively, the amount of the titanium source solution charged in step II(a) accounts for 20 to 40 % by volume of the total amount of the titanium source solution charged in the method, and the amount of the titanium source solution charged in step II(c) accounts for 60 to 80 % by volume of the total amount of the titanium source solution charged in the method.

A ratio of the mixed alkali solution charged in steps II(a) and 11(b) can be in a range of from 2:1 to 1:2, or from 1.5:1 to 1:1. Alternatively, the amount of the mixed alkali solution charged in step II(a) accounts for 33 to 67 vol%, and preferably 50 to 60 vol%, and the amount of the mixed alkali solution charged in step 11(b) accounts for 33 to 67 vol%, and preferably 40 to 50 vol%, of the total amount of the mixed alkali solution charged in steps II(a) and 11(b).

In some embodiments of the method of the invention, in step IV, the precipitate washing process comprises washing with deionized water until acid ions cannot be detected, the drying temperature is from 100°C to 120°C, the drying time is from 4°C to 12h, and the calcination temperature is from 800°C to 1000°C.

The composite oxide prepared according to the method provided by the invention comprises 5 wt% to 21wt% of titanium dioxide, and the titanium dioxide and alumina are uniformly mixed. Furthermore, the inventors have surprisingly found that the composite oxide has a surface micromorphology of coral thicket 3D layered structure. The coral-like 3D layered structure can make the composite oxide have a higher specific surface area, thereby having more active sites and stronger electron capture ability, which helps to promote the co-catalytic effect of TiO₂ as an electron promoter, improving the overall performance of the catalyst including activity and selectivity.

In addition, it is generally deemed that during the calcination of TiO₂, rutile will be formed starting from a calcination temperature of 500°C, and most of the crystal phase of TiO₂ changes from anatase phase to rutile phase at a calcination temperature above 700°C. However, after the composite oxide prepared by the method provided herein has been calcined at a high temperature of 800°C or higher, it has been found through X-ray diffraction analysis that anatase-type titanium dioxide accounts for 95 to 100 wt% of all crystal phases of TiO₂, contrary to common sense. It is believed that TiO₂ present in the form of anatase in the composite oxide has an electron induction effect and can effectively induce a decrease in electron cloud density of the active metal Ni in the prepared catalyst (compared with the rutile phase generated by high-temperature calcination), thereby enhancing the adsorption ability to alkynes in the reaction raw materials and improving the selective hydrogenation activity and selectivity of the catalyst.

In a third aspect, the present invention further provides use of a composite oxide, including use of the composite oxide according to the first aspect of the invention or the composite oxide prepared by the method according to the second aspect as a catalyst carrier.

According to the invention, the type of the catalyst is not particularly limited.

In some embodiments, the present invention provides a hydrogenation catalyst comprising the composite oxide according to the first aspect of the invention or the composite oxide prepared by the method according to the second aspect, and an active component such as palladium, nickel, iron or cobalt.

In some embodiments of the hydrogenation catalyst of the invention, the hydrogenation metal is nickel. In some embodiments, the hydrogenation catalyst may have a nickel content of 8-25 wt%, and preferably 12-20 wt%. In some other embodiments, the hydrogenation catalyst comprises palladium, which may be present in an amount of 0.1-2 wt%, for example 0.15-1.5 wt%.

In a fourth aspect, the present invention further provides a process for alkyne selective hydrogenation, comprising subjecting a distillate oil to selective hydrogenation of alkynes in the presence of the hydrogenation catalyst described in the third aspect to increase the production of butadiene, wherein the distillate oil includes C4 distillate oil, preferably a high-alkyne tail gas co-produced in a butadiene extraction unit.

Further, in the process for selective hydrogenation of alkynes, a reaction temperature is from 20°C to 40°C, a molar ratio of hydrogen to alkyne is from 1:1 to 2.5:1, a pressure is from 0.5MPa to 0.8MPa, and a circulation ratio is from 10 : 1 to 30:1.

The catalyst provided by the invention exhibits a relatively high vinyl acetylene conversion rate and a high selectivity to 1,3-butadiene in the selective hydrogenation of alkynes.

### Brief description of the drawings

Fig. 1 shows a coral thicket-like 3D microscopic morphology of the alumina-titanium dioxide composite oxide prepared in Example 1.
Fig. 2 shows an XRD pattern of the alumina-titanium dioxide composite oxide prepared in Example 1.

### Examples

In order to make the invention easy to be understood, the invention will be described in detail below in conjunction with examples. These examples are only for illustrative purposes and do not limit the scope of application of the invention.

Unless otherwise indicated, the raw materials or components used in the invention can be commercially obtained or prepared by a conventional method.

An ASAP 2020 model adsorption instrument (N₂ adsorption and desorption method) available from the Mike Instrument Company, USA was used to determine the specific surface area and pore structure of the composite oxide. Prior to measuring, a sample of the composite oxide was degassed at 623K for 4 hours, and nitrogen was adsorbed at liquid nitrogen temperature. AMSM software was used to process the sample data, and the Brunauer-Emmet-Teller (BET) method was used to obtain the specific surface area of the sample. The Barrett-Joyner-Halenda (BJH) method was used to obtain the average pore diameter based on the nitrogen adsorption isotherm curve, and the P/Po single-point desorption curve was used to obtain the pore volume.

A QUANTA 200 model scanning electron microscope available from the FEI Company was used to observe the morphology of the composite oxide.

The crystal phase structure of the composite oxide was characterized by using an EMPYREAN X-ray diffractometer available from PANalytical B.V., the Netherlands. Cu Kα is used as the radiation source, X-ray tube voltage is 40kV, light tube current is 40mA, slit width is 10mm, scanning range is 5-90°, and scanning speed is 0.013°/s.

### Example 1

328.02 g of Al₂(SO₄)₃ was dissolved in deionized water to prepare 1000 mL of aluminum sulfate solution. 21.14 g of TiO(OH)₂ was dissolved in 16 mL of 98 wt% sulfuric acid, and deionized water was added thereto to prepare 500 mL of metatitanic acid solution in dilute sulfuric acid. 18 g of NH₄HCO₃ was dissolved in 600 mL of deionized water to prepare an ammonium bicarbonate solution, then 250 mL of 27 wt% ammonia water was added thereto, and the resulting mixed solution was stirred to mix uniformly and then complemented with deionized water to prepare 1000 mL of mixed alkali solution.

Under normal pressure and at a temperature of 55°C, 230 mL of the metatitanic acid solution in dilute sulfuric acid and 360 mL of the mixed alkali solution were co-current added into 1000 mL of the prepared aluminum sulfate solution with strong stirring, and the pH value of the resulting mixed solution was maintained at 3 to 4 for 15 min. Then, additional 320 mL of the mixed alkali solution was added to bring the pH value to 9 to 10, and the reaction mixture was maintained at that pH value for 15 min. Then, the remaining metatitanic acid solution in dilute sulfuric acid was added thereto to bring the pH value to 7.5 to 8.5, and the reaction mixture was maintained at that pH value for 6 to 10 minutes. Then, the temperature was raised to 92°C and maintained for 20 minutes, and the reaction mixture was then filtered. The filter cake was repeatedly washed with 20 times volume of deionized water 5 times, and the washed filter cake was dried at 110°C for 6 hours and calcined at 850°C for 5 hours. 114.3 g of composite oxide with a TiO₂ content of 15.0 wt% was obtained.

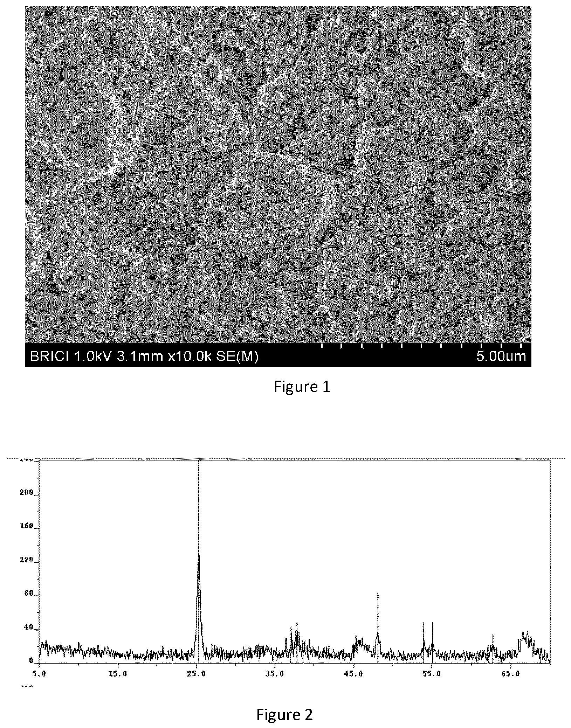

It can be seen from the SEM in Fig. 1 that the composite oxide exhibits a surface micromorphology of coral thicket-like 3D layered structure. Fig. 2 shows the XRD pattern of the alumina-titanium dioxide composite oxide prepared in Example 1. For titanium dioxide, only characteristic diffraction peak of anatase appears at 2θ=25.4° and no diffraction peaks of other crystal phases appear at other positions, indicating that all TiO₂ component in the composite oxide is present as the anatase crystal phase. In other words, the titanium dioxide in the anatase crystal phase accounts for 100 wt% of the total titanium dioxide.

### Example 2

256.04 g of Al₂(SO₄)₃ was dissolved in deionized water to prepare 1000 mL of aluminum sulfate solution. 32.15 g of Ti(SO₄)₂ was dissolved in deionized water, and 5 mL of concentrated sulfuric acid (98 %) was added thereto, to prepare 500 mL of titanium sulfate solution in dilute sulfuric acid. 18 g of NH₄HCO₃ was dissolved in 600 mL of deionized water to prepare an ammonium bicarbonate solution, then 250 mL of 27 wt% ammonia water was added thereto, and the resulting mixed solution was stirred to mix uniformly and then complemented with deionized water to prepare a 1000 mL mixed alkali solution.

Under normal pressure and at a temperature of 60°C, 280 mL of the titanium sulfate solution in dilute sulfuric acid and 320 mL of the mixed alkali solution were co-current added into 1000 mL of the aluminum sulfate solution with strong stirring, and the pH value of the resulting mixed solution was maintained at 3 to 4 for 15 min. Then, additional 240 mL of the mixed alkali solution was added to bring the pH value to 9 to 10, and the reaction mixture was maintained at that pH value for 15 min. Then, the remaining titanium sulfate solution in dilute sulfuric acid was added thereto to bring the pH value to 7.5 to 8.5. The temperature was raised to 85°C and maintained for 40 minutes, and the reaction mixture was then filtered. The filter cake was repeatedly washed with 20 times volume of deionized water 5 times, and the washed filter cake was dried at 110°C for 6 hours and calcined at 950°C for 5 hours. 86.8 g of alumina-titanium dioxide composite oxide with a TiO₂ content of 12.3 % was obtained.

### Example 3

The procedure for preparing titanium dioxide-alumina composite oxide as described in Example 1 was repeated, except that 379.79 g of Al(NO₃)₃ was dissolved in deionized water to prepare 1000 mL of aluminum nitrate solution and that 25.99 g of Ti(OCH₂CH₃)₄ was dissolved in absolute ethanol to prepare 500 mL of tetraethyl titanate solution in ethanol. Finally, alumina-titanium dioxide composite oxide with a TiO₂ content of 9.1 % was obtained.

### Example 4

The procedure for preparing titanium dioxide-alumina composite oxide as described in Example 1 was repeated, except that 396.92 g of Al(NO₃)₃ was dissolved in deionized water to prepare 1000 mL of aluminum nitrate solution and that 6.13 g of TiO(OH)₂ was dissolved in sulfuric acid and deionized water was added thereto to prepare 500 mL of metatitanic acid solution in dilute sulfuric acid. Finally, alumina-titanium dioxide composite oxide with a TiO₂ content of 5.0 % was obtained.

### Example 5

The procedure for preparing titanium dioxide-alumina composite oxide as described in Example 1 was repeated, except that 396.92 g of Al(NO₃)₃ was dissolved in deionized water to prepare 1000 mL of aluminum nitrate solution, that 6.13 g of TiO(OH)₂ was dissolved in sulfuric acid and deionized water was added thereto to prepare 500 mL of metatitanic acid solution in dilute sulfuric acid, and that the temperature for calcining the filter cake was 800°C. Finally, alumina-titanium dioxide composite oxide with a TiO₂ content of 5.0 % was obtained.

### Example 6

The procedure for preparing titanium dioxide-alumina composite oxide as described in Example 1 was repeated, except that 374.13 g of AlCl₃ ·6H₂O was dissolved in deionized water to prepare 1000 mL of aluminum chloride solution and that 59.99 g of Ti(OCH₂CH₃)₄ was dissolved in absolute ethanol to prepare 500 mL of tetraethyl titanate solution in ethanol. Finally, alumina-titanium dioxide composite oxide with a TiO₂ content of 21.0% was obtained.

Scanning electron microscopy and XRD test results showed that the composite oxides prepared in Examples 2-6 were similar to that of Example 1, exhibiting a surface micromorphology of coral thicket-like 3D layered structure, and that all TiO₂ component in the composite oxides was present as the anatase crystal phase.

### Comparative example 1

268.46 g of Al₂(SO₄)₃ was dissolved in deionized water to prepare 1000 mL of aluminum sulfate solution. 24.5 g of TiO(OH)₂ was dissolved in sulfuric acid, and deionized water was added thereto to prepare 1000 mL of metatitanic acid solution in dilute sulfuric acid. 18 g of NH₄HCO₃ was dissolved in 600 mL of deionized water to prepare an ammonium bicarbonate solution, then 250 mL of 24-28 wt% ammonia water was added thereto, and the resulting mixed solution was stirred to mix uniformly and then complemented with deionized water to prepare 1000 mL of mixed alkali solution.

Under the conditions of normal pressure and a temperature of 70-75°C, the above three solutions, i.e., the aluminum sulfate solution in deionized water, the metatitanic acid solution in dilute sulfuric acid and the mixed alkali solution, were co-current combined to conduct co-precipitation. The flow rate of the mixed alkali solution was controlled so as to keep the pH value of the precipitating reactants within a range of 5.0 to 6.0 for 8 minutes, then the flow rate of the mixed alkali solution was increased so as to keep the pH value of the combined solution within a range of 8.5 to 9.5 for 8 minutes, then the flow rate of the mixed alkali solution was reduced so as to keep the pH value of the combined solution within a range of 5.0 to 6.0 for 8 minutes, and then the flow rate of the mixed alkali solution was increased so as to keep the pH value of the precipitating reactants within a range of 8.5 to 9.5. The operations were so repeated until the dropwise addition of the solutions A1 and B1 were completed. The reaction liquid was left to stand for 30 minutes at 70°C and then filtered, and the filter cake was washed with 15 times volume of deionized water for 30 minutes, filtered again, and washed again. This process was repeated four times, and finally the filter cake was dried at 100-120°C for 8-12 hours and calcined at 950°C for 5 hours to afford a titanium dioxide-alumina composite. The structure and performance results of the composite are shown in Table 1.

### Comparative example 2

401.88 g of analytically pure AlCl₃·6H₂O was dissolved in 1000 ml of deionized water to prepare solution A1; 43.25 g of chemically pure Ti(OCH₂CH₃)₄ was dissolved in 500 ml of benzene (having a benzene content of 99.8 wt%) to prepare solution B1; 18 g of analytically pure NH₄HCO₃ was dissolved in 600 ml of deionized water, 250 ml of 24-28 wt% ammonia water was added thereto, and the resulting mixed solution was stirred to mix uniformly and then complemented with deionized water to prepare 1000 mL of solution C1.

Under the conditions of normal pressure and a temperature of 70-75°C, the three solutions, A1, B1 and C1, were co-current combined to conduct co-precipitation. The flow rate of the solution C1 was controlled so as to keep the pH value of the precipitating reactants within a range of 5.0 to 6.0 for 8 minutes, then the flow rate of the solution C1 was increased so as to keep the pH value of the combined solution within a range of 8.5 to 9.5 for 8 minutes, then the flow rate of the solution C1 was reduced so as to keep the pH value of the combined solution within a range of 5.0 to 6.0 for 8 minutes, and then the flow rate of the solution C1 was increased so as to keep the pH value of the precipitating reactants within a range of 8.5 to 9.5. The operations were so repeated until the dropwise addition of the solutions A1 and B 1 were completed. The reaction liquid was left to stand for 30 minutes at 70°C and then filtered, and the filter cake was washed with 15 times volume of deionized water for 30 minutes, filtered again, and washed again. This process was repeated four times, and finally the filter cake was dried at 100-120°C for 8-12 hours and calcined at 550°C for 5 hours to afford 42.7 g of a titanium dioxide-alumina composite. The structure and performance results of the composite are shown in Table 1.

### Comparative example 3

The procedure for preparing titanium dioxide-alumina composite oxide as described in Comparative Example 2 was repeated, except that the calcining temperature after drying was 950°C.

Scanning electron microscopy and XRD test results showed that the composite oxides prepared in Comparative Examples 1-3 did not have a coral thicket-like 3D layered structure, and the TiO₂ crystal phase therein is mainly rutile crystal phase.

For the carriers prepared above, the N₂ adsorption-desorption method was used to determine the specific surface area and pore structure of the composite oxides.

**Table 1. Analytical data of titanium dioxide-alumina composites**

| | TiO₂ content | 'BET specific surface area X (m²/g) | Pore volume Z (mL/g) | Average pore diameter Y (nm) | X/Y | X/Z |
|---|---|---|---|---|---|---|
| Example 1 | 15.0 wt% | 110 | 0.39 | 15.6 | 7.05 | 282 |
| Example 2 | 12.3 wt% | 112 | 0.38 | 15.4 | 7.27 | 295 |
| Example 3 | 9.1 wt% | 120 | 0.38 | 15.7 | 7.64 | 316 |
| Example 4 | 5.0 wt% | 130 | 0.39 | 15.8 | 8.23 | 333 |
| Example 5 | 5.0 wt% | 153 | 0.48 | 13.6 | 11.25 | 319 |
| Example 6 | 21.0 wt% | 105 | 0.38 | 14.8 | 7.09 | 276 |
| Comp. Example 1 | 20.0 wt% | 55 | 0.31 | 15.0 | 3.67 | 177 |
| Comp. Example 2 | 15.14 wt% | 286 | 0.54 | 7.5 | 38.1 | 530 |
| Comp. Example 3 | 15.14wt% | 64 | 0.32 | 14.9 | 4.30 | 200 |

**Table 2. Pore distribution of titanium dioxide-alumina composite support**

| | Proportion of pores with a pore diameter in a range of 10 to 20 nm accounting for all pores, on volume basis |
|---|---|
| Example 1 | 86% |
| Example 2 | 88% |
| Example 3 | 89% |
| Example 4 | 90% |
| Example 5 | 85% |
| Example 6 | 85% |
| Comp. Example 1 | 78% |
| Comp. Example 2 | 34% |
| Comp. Example 3 | 82% |

### Catalyst preparation

### Example 7

156 g of the composite oxide prepared in Example 1 was impregnated with 100 mL of aqueous solution of nickel nitrate having a concentration of 24.72 g Ni/100mL for 1h. After filtering, the filer cake was dried at 110°C for 6h and then calcined at 600°C for 4h to afford Ni/Al₂O₃-TiO₂ catalyst A having a Ni content of 13.68 %.

### Example 8

180 g of the composite oxide prepared in Example 1 was impregnated with 100 mL of aqueous solution of nickel nitrate having a concentration of 20.0 g Ni/100mL for 0.5h. After filtering, the filer cake was dried at 110°C for 5h and then calcined at 550°C for 5h. Next, 100 g of the above-prepared calcined catalyst precursor was impregnated with 100 mL of aqueous solution of nickel nitrate having a concentration of 12.24 g Ni/100mL for 0.5h. After filtering, the filer cake was dried at 110°C for 4h and then calcined at 550°C for 6h to afford Ni/Al₂O₃-TiO₂ catalyst B having a Ni content of 19.82%.

### Example 9

156 g of the composite oxide prepared in Example 6 was impregnated with 100 mL of aqueous solution of nickel nitrate having a concentration of 13.56 g Ni/100mL for 1h. After filtering, the filer cake was dried at 110°C for 6h and then calcined at 600°C for 4h to afford Ni/Al₂O₃-TiO₂ catalyst C having a Ni content of 8 %.

### Example 10

180 g of the composite oxide prepared in Example 4 was impregnated with 100 mL of aqueous solution of nickel nitrate having a concentration of 20.0 g Ni/100mL for 0.5h. After filtering, the filer cake was dried at 110°C for 5h and then calcined at 550°C for 5h. Next, 100 g of the above-prepared calcined catalyst precursor was impregnated with 100 mL of aqueous solution of nickel nitrate having a concentration of 20 g Ni/100mL for 0.5h. After filtering, the filer cake was dried at 110°C for 4h and then calcined at 550°C for 6h to afford Ni/Al₂O₃-TiO₂ catalyst D having a Ni content of 25%.

### Example 11

180 g of the composite oxide prepared in Example 2 was impregnated with 100 mL of aqueous solution of nickel nitrate having a concentration of 20.0 g Ni/100mL for 0.5h. After filtering, the filer cake was dried at 110°C for 5h and then calcined at 550°C for 5h. Next, 100 g of the above-prepared calcined catalyst precursor was impregnated with 100 mL of aqueous solution of nickel nitrate having a concentration of 12.24 g Ni/100mL for 0.5h. After filtering, the filer cake was dried at 110°C for 4h and then calcined at 550°C for 6h to afford Ni/Al₂O₃-TiO₂ catalyst E having a Ni content of 19.82%.

### Example 12

180 g of the composite oxide prepared in Example 3 was impregnated with 100 mL of aqueous solution of nickel nitrate having a concentration of 20.0 g Ni/100mL for 0.5h. After filtering, the filer cake was dried at 110°C for 5h and then calcined at 550°C for 5h. Next, 100 g of the above-prepared calcined catalyst precursor was impregnated with 100 mL of aqueous solution of nickel nitrate having a concentration of 12.24 g Ni/100mL for 0.5h. After filtering, the filer cake was dried at 110°C for 4h and then calcined at 550°C for 6h to afford Ni/Al₂O₃-TiO₂ catalyst F having a Ni content of 19.82%.

### Example 13

156 g of the composite oxide prepared in Example 5 was impregnated with 100 mL of aqueous solution of nickel nitrate having a concentration of 24.72 g Ni/100mL for 1h. After filtering, the filer cake was dried at 110°C for 6h and then calcined at 600°C for 4h to afford Ni/Al₂O₃-TiO₂ catalyst G having a Ni content of 13.68 %.

### Example 14

100 g of the composite oxide prepared in Example 1 was immersed into 85 mL of aqueous solution of palladium chloride having a palladium content of 0.32 g/100mL. After 1.5 hours, the impregnated composite carrier was filtered out, reduced with 120 mL of aqueous solution of hydrazine hydrate with a concentration of 10 wt% at room temperature for 1 hour, rinsed repeatedly with deionized water until the chloride ions were washed off. After draining, the solids were dried at 120°C for 6 hours and then calcined at 480°C for 4 hours to afford Pd/Al₂O₃-TiO₂ catalyst H having a Pd content of 0.3%.

### Comparative example 4

156 g of the composite oxide prepared in Comparative Example 1 was impregnated with 100 mL of aqueous solution of nickel nitrate having a concentration of 24.72 g Ni/100mL for 1h. After filtering, the filer cake was dried at 110°C for 6h and then calcined at 600°C for 4h to afford Ni/Al₂O₃-TiO₂ catalyst I having a Ni content of 13.68 %.

### Comparative example 5

156 g of the composite oxide prepared in Comparative Example 2 was impregnated with 100 mL of aqueous solution of nickel nitrate having a concentration of 24.72 g Ni/100mL for 1h. After filtering, the filer cake was dried at 110°C for 6h and then calcined at 600°C for 4h to afford Ni/Al₂O₃-TiO₂ catalyst J having a Ni content of 13.68 %.

### Comparative example 6

156 g of the composite oxide prepared in Comparative Example 3 was impregnated with 100 mL of aqueous solution of nickel nitrate having a concentration of 24.72 g Ni/100mL for 1h. After filtering, the filer cake was dried at 110°C for 6h and then calcined at 600°C for 4h to afford Ni/Al₂O₃-TiO₂ catalyst K having a Ni content of 13.68 %.

### Example 15

This example demonstrated the application of catalysts in the selective hydrogenation of butadiene extraction tail gas.

The catalysts used in this example were the catalysts A-K.

The raw material used in this example was butadiene extraction tail gas from a certain plant. The composition is shown in Table 3.

**Table 3. Composition of the butadiene extraction tail gas from a certain plant**

| No. | Composition | Wt% |
|---|---|---|
| 1 | Isobutane | 2.658 |
| 2 | n-butane | 5.652 |
| 3 | trans-2-butene | 5.994 |
| 4 | n-butene | 24.635 |
| 5 | Isobutylene | 31.445 |
| 6 | cis-2-butene | 2.986 |
| 7 | 1,3-butadiene | 3.162 |
| 8 | 1,2-butadiene | 0 |
| 9 | Vinyl acetylene (VA) | 20.563 |
| 10 | Ethyl acetylene (EA) | 2.344 |

In this example, a fixed-bed small-scale evaluation device from Tuochuan Scientific Research Equipment Co., Ltd. was used, and 50 mL of the catalyst was loaded to perform the selective hydrogenation of the butadiene extraction tail gas.

Reaction conditions include reaction pressure of 0.5 to 0.7 MPa, hydrogen amount of 1.92 L/h, reactor inlet temperature of 25°C, circulation ratio of 20:1, and raw material feeding amount of 25 mL/h.

Catalysts A to K were evaluated under the same conditions, and the results of hydrogenation to remove alkynes are shown in Table 4.

**Table 4. Selective hydrogenation results of butadiene extraction tail gas**

| Catalyst | 1,3-Butadiene (product) % | Vinyl acetylene (product) | 1-Butyne (product) | Conversion of vinyl acetylene | Selectivity to 1,3-butandiene * * (%) |
|---|---|---|---|---|---|
| A | 10.529 | 3.964 | 0.804 | 80.72 | 44.38 |
| B | 9.536 | 3.35 | 0.737 | 83.71 | 37.50 |
| E | 9.583 | 3.849 | 0.759 | 81.28 | 38.42 |
| F | 9.820 | 3.971 | 0.762 | 80.69 | 40.13 |
| G | 10.271 | 3.355 | 0.727 | 83.68 | 41.32 |
| H | 10.508 | 3.105 | 0.687 | 84.90 | 42.08 |
| C | 10.519 | 4.458 | 0.845 | 78.32 | 45.68 |
| D | 9.055 | 4.065 | 0.811 | 80.23 | 35.72 |
| I | 8.498 | 8.425 | 1.171 | 66.32 | 43.96 |
| J | 4.615 | 0.757 | 0.512 | 96.32 | 7.34 |
| K | 8.522 | 8.155 | 1.463 | 60.34 | 43.20 |

| | | | | | |
|---|---|---|---|---|---|
| ** Selectivity to 1,3-butadiene = (1,3-butadiene in the product - 1,3-butadiene in the raw material) / (vinyl acetylene in the raw material - vinyl acetylene in the product) | | | | | |

It can be seen from the above table that the catalysts provided by the invention exhibit a high conversion of vinyl acetylene and a high selectivity to 1,3-butadiene. Because the vinyl acetylene in the hydrogenated product can be controlled to a lower level, the hydrogenated product can be directly returned to the extraction system to increase butadiene production.

### Example 16

A long-term stability experiment was conducted by using the catalyst A prepared in Example 7 under the same conditions as in Example 15. The stability evaluation experimental data for 1000 hours are shown in Table 5.

**Table 5**

| Run time (h) | Selectivity to 1,3-butadiene (%) | Conversion of vinyl acetylene (%) |
|---|---|---|
| 96 | 44.38 | 80.72 |
| 192 | 43.65 | 82.74 |
| 288 | 42.97 | 81.62 |
| 384 | 42.12 | 81.53 |
| 480 | 43.38 | 81.08 |
| 576 | 44.85 | 80.45 |
| 672 | 44.38 | 80.49 |
| 768 | 43.57 | 81.27 |
| 864 | 44.81 | 80.72 |
| 960 | 42.76 | 81.67 |
| 1056 | 42.59 | 81.76 |

It can be seen from Table 5 that the catalyst provided by the invention exhibits a high stability in the selective hydrogenation of butadiene tail gas with a high alkyne content as a feedstock. The hydrogenation catalyst of the invention not only has a high selectivity to 1,3-butadiene (%) and a high vinyl acetylene conversion (%), but also can maintain the selectivity and the conversion at a high level for a long time, so that it is suitable for long-term operation.

It should be noted that the above-described embodiments are only used to explain, but do not constitute any limitation on, the invention. The invention has been described with reference to exemplary embodiments, but it should be understood that the words used therein are descriptive and explanatory rather than restrictive. The invention may be modified as specified within the scope of the claims of the invention and may be modified without departing from the scope and spirit of the invention. Although the invention described herein relates to specific methods, materials and embodiments, it is not intended that the invention be limited to these specific examples disclosed herein, but rather the invention extends to all other methods and applications having the same functions.

## Claims

1. A composite oxide, comprising 60 to 95 wt% of aluminum oxide and 5 to 40 wt% of titanium dioxide, wherein the composite oxide has a specific surface area expressed as X m²/g as measured by a BET method and an average pore diameter expressed as Y nm as measured by a nitrogen adsorption isotherm method, with a ratio of X to Y being in a range of from 5 to 30, wherein in the composite oxide, titanium dioxide in an anatase crystal phase accounts for 95 wt% to 100 wt% of the total titanium dioxide, as measured by X-ray diffraction method, and wherein X is in a range of from 50 to 200, preferably from 60 to 180, and more preferably from 80 to 150, and Y is in a range of from 5 to 25 nm.

2. The composite oxide according to claim 1, wherein the composite oxide has a pore volume expressed as Z mL/g, wherein Z is in a range of from 0.3 to 0.5, and wherein a ratio of X to Z is from 220 to 400, and preferably from 250 to 350.

3. The composite oxide according to claim 1 or 2, having at least one of the following features:
- X is from 90 to 150;
- Y is from 9 to 20, and preferably from 12 to 16;
- at least 85% of pores have a pore diameter in a range of 10 to 20 nm;
- Z is from 0.3 to 0.4; and/or the composite oxide comprises 5 wt% to 21 wt% of titanium dioxide; and
- the composite oxide has a coral thicket-like 3D layered structure.

4. A method for preparing a composite oxide, comprises the following steps:
I, dissolving a soluble aluminum source in water to form an aluminum source solution, dissolving a titanium source in an acid solution to form a titanium source solution, and mixing an ammonium salt and an alkali liquid to form a mixed alkali solution;
II, (a) adding the titanium source solution and the mixed alkali solution to the aluminum source solution, and maintaining a resulting mixed solution at a first pH value for a first period of time; (b) adding an additional amount of the mixed alkali solution to the mixed solution, and maintaining a resulting mixed solution at a second pH value for a second period of time; (c) adding an additional amount of the titanium source solution to the mixed solution, and maintaining a resulting mixed solution at a third pH value for a third period of time;
III, after step II(c), raising the temperature of the mixed solution and maintaining that temperature for a fourth period of time to obtain a precipitate; and
IV, drying and calcining the precipitate to obtain a composite oxide comprising alumina and titanium dioxide, with washing and filtering being preferably conducted prior to the drying.

5. The method according to claim 4, wherein in step II, the first pH value is less than 5, and preferably 3 to 4, the second pH value is greater than 8.5, and preferably 9 to 10, and the third pH value is greater than 7 and less than 9, and preferably 7.5 to 8.5.

6. The method according to claim 4 or 5, wherein each of the first, second and third periods of time is from 5 to 20 minutes, and preferably from 10 to 15 minutes; and/or the fourth period of time is from 20 to 60 minutes.

7. The method according to any one of claims 4 to 6, wherein in step II, an operating temperature is 25°C to 60°C; and/or in step III, the temperature is raised to 80°C to 150°C; and/or in step IV, a drying temperature is 110°C to 130°C, and/or a calcining temperature is 800°C to 1000°C.

8. Use of the composite oxide according to any one of claims 1 to 3 or the composite oxide prepared according to the method of any one of claims 4 to 7 as a catalyst carrier.

9. A hydrogenation catalyst, comprising the composite oxide of any one of claims 1-3 or the composite oxide prepared according to the method of any one of claims 4-7 and an active component, preferably the active component is nickel, and preferably the catalyst has a nickel content of 8 to 25 wt%, and more preferably 12 to 20 wt%.

10. A process for selective hydrogenation of alkynes, comprising subjecting a distillate oil to selective hydrogenation of alkynes in the presence of the hydrogenation catalyst of claim 9 to increase the production of butadiene, wherein the distillate oil comprises a C4 distillate oil, preferably a high-alkyne tail gas co-produced in a butadiene extraction unit; preferably, in the process of selective hydrogenation of alkynes, a reaction temperature is 20°C to 40°C, a molar ratio of hydrogen to alkynes is 1: 1 to 2.5:1, a pressure is 0.5MPa to 0.8MPa, and a circulation ratio is 10:1 to 30:1.
